# EUROPEAN PATENT SPECIFICATION

(11) **EP 1 276 459 B1**
(45) Date of publication and mention of the grant of the patent: **30.08.2006**
(21) Application number: 01929678.9
(22) Date of filing: 19.04.2001
(51) Int. Cl.: A61K 8/44, A61K 8/67, A61Q 19/00

(54) **COSMETIC OR DERMATOLOGICAL CREAM COMPOSITION, THE PREPARATION AND THE USE THEREOF**
KOSMETISCHE ODER DERMATOLOGISCHE CREMEZUBEREITUNG, DEREN HERSTELLUNG UND VERWENDUNG
COMPOSITION COSMETIQUE OU DERMATOLOGIQUE EN CREME, SA PREPARATION ET SON UTILISATION

(30) Priority: 20.04.2000 FI 20000956
(43) Date of publication of application: 22.01.2003
(73) Proprietor: Rasi, Simo, 90220 Oulo (US)
(72) Inventor: Rasi, Simo, 90220 Oulo (US)
(74) Representative: Hjelt, Pia Dorrit Helene
(86) International application number: PCT/FI2001/000383
(87) International publication number: WO 2001/080819

(56) References cited:
- US-A- 3 544 684
- US-A- 4 839 159

## Description

The invention relates to a later on defined cosmetic and dermatological cream composition improving the absorption of the active ingredients and containing vitamins B_{T} i.e. carnitine and B₁₃ i.e. orotic acid, the production and the use thereof.

Carnitine derivatives as such or as bound have been used in dermatological compositions and treatments. Patent publications US 5,639,767, US 5,637,305 and US 5,627,212 enclose examples of such treatments. Patent publications utilize medical compounds prepared from O-esters and aromatic acids of L-carnitine, acid azelates of L-camitine, esters of L-carnitine and acyl carnitine as well as hydroxycarboxyl acids for treatment of dermatoses, like psoriasis, and for cosmetic use. In all these treatments and ways of using carnitine is bound or as a compound, not as free carnitine. It is, on the other hand, known from the patent publication US 5,008,288 to use carnitine derivatives and cysteic acid as carriers when they are bound to pharmaceutically active compounds in order to bring these compounds to the desired sites in the body, like limb muscles or heart. Although the ability of carnitine derivatives to conduct pharmaceutically active agents in cells is known, the use of carnitine as a carrier of active ingredients of dermatological cream compositions is not known. US 4,839,359 discloses a composition comprising L-carnitine in a suitable vehicle.

Whey, especially colostrum whey, has been used as a dermatological product (GB 2052979). Although orotic acid is mentioned as a nutritionally important component of whey-based beverages, the importance of orotic acid as a dermatologically active ingredient is not known. The dermatological effects of whey and especially colostrum whey has been seen to result from pH, lactic acid bacteria, growth factors and vitamins such as B₁, B₂, B₁₂, nicotinic acid, pantothenic acid, biotin and folic acid. US3,544,684 discloses a cosmetic composition containing orotic acid.

In dermatological cream compositions it is important to find such a composition, which contains an effective cell health promoting effect and which has a cell rejuvenating effect. Furthermore is important that this cell condition promoting active ingredient is carried to a target cell.

The present invention describes such an effective combination, which provides skin health promoting effect and at the same time an efficient migration to the target cells. Characteristics of the cream composition according to the invention, the production and the use thereof have been presented in the claims.

Figure 1 discloses schematically the proportion of carnitine and acylcarnitine in the transition of fatty acids to the mitochondria.

Figure 2 discloses schematically the formation reaction of orotate and the contribution of the mitochondrial enzyme i.e. dihydro-orotate dehydrogenase to this reaction.

Figure 3 discloses schematically chaplet-like cream compositions of different colors in a gel.

Next the invention is described in detail. The terms used in the specification and in the claims have the same meaning as they usually have in dermatology, biochemistry and production technology of dermatologically active cream compositions, but some of the terms are used in this context somewhat differently and these broader or differently interpreted terms are explained more precisely in the following.

"Active ingredient" means a compound, which promotes the migration of other small molecules, like orotate, through the cell membranes into the cell and promotes the return or recovery of the cells as well as prevents them from ageing.

"Absorption" means the migration of the active ingredients e.g. from the surface of a skin through the walls of cell membranes and cell organelles into the cells below the surface.

"Cosmetic" means agents and operations used in cosmetology and hygiene for a healthy skin. Cosmetic agents may e.g. in respect of skin cells help the skin to stay vital and elastic longer.

"Dermatological" means agents or operations used for treating different dermatoses, like psoriasis and atopic rash, and skin lesions, like leg ulcers. Dermatological products are used e.g. to expedite recovery of injured cells as well as for returning the elasticity.
"Cream composition" means a composition, which may contain in addition to conventional basic cream compositions different cosmetic or dermatological active agents. Such cream compositions have been described for example in patent publications EP 577 516, EP 705 593 and EP 716 849.

"Basic cream composition" means a cream composition whereto necessary compounds may be added in order to prepare a dermatological or cosmetic cream composition. A basic cream composition contains tested cream compositions generally used in dermatological products.

"Gel" means a salvelike composition, which however does not contain grease. A gel may contain same active ingredients and/or compounds protecting active agents as a grease containing cream composition. A gel may contain for example hyaluronic acid or its polymers or methylcellulose derivatives.

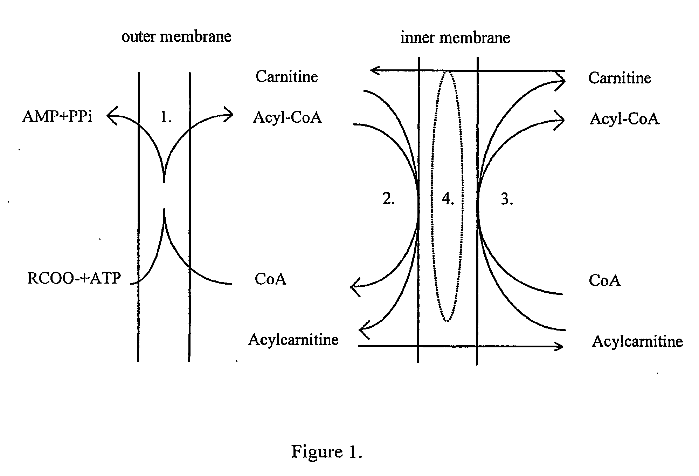

"Vitamin B_{T} i.e. carnitine" means an agent, which transfers long chain fatty acids through the inner membrane of a mitochondrion to the energy production occurring with the help of β-oxidation (Figure 1). Carnitine was found already in the beginning of the century. The chemical name of carnitine is 3-carboxy-2-hydroxy-N,N,N-trimethyl-1-propanamium-hydroxide and its formula is (CH₃)₃N⁺CH₂CH-(OH)CH₂COO⁻. The name vitamin B_{T} comes from the name of a *Tenebrio molitor* -worm; carnitine is of the growth factors of the worm.

"Vitamin B₁₃ i.e. orotic acid" means ia. a vitamin existing in whey. Milk whey, which is called "oros" in Greek (in Latin "serum lactis"), has been used possibly already centuries for the production of whey beverages. The name of the milk whey in Greek is also shown in the name "orotic acid" i.e. orotate. Only cow milk contains orotic acid (about 80 mg/l), which is also known by the name vitamin B₁₃. Orotic acid is a nutritionally important component (Sienkiewicz and Riedel, 1990, Whey and whey utilization, Possibilities for utilization in agriculture and foodstuffs production p. 26-39, 85-88, 165-205, 231-238, 275-277 320-328, 2. edition Gelsenkirhen-Buer, Germany (1990). Verlag Th. Mann 379 p.), which is almost completely dissolved in whey.

A cream composition according to the invention has remarkable benefits when compared to the known prior art. The combination of L-carnitine and orotate achieves surprising synergistic ef fects. L-carnitine used as an active ingredient in the cream composition is able to migrate through the walls of cell membranes and cell organelles and helps the migration of other small molecules, like orotate added into the composition, through the cell membranes into the cell. Orotic acid expedites the recovery of injured cells, which means in case of skin cells, that the skin remains vital longer as well as that unhealthy skin recovers faster and returns the elasticity.

The present invention is based on the action of carnitine in mitochondria and its migration through the membranes as shown in Figure 1. In Figure 1 (1) means acyl-CoA-synthetase, (2) means outer carnitine acyl transferase, (3) means inner carnitine acyl transferase and (4) means carnitine/acyl carnitine translocase. In Figure 1 abbreviation AMP means adenosine triphosphate, abbreviation CoA means coenzyme A, abbreviation Ppi means inorganic pyrophosphate and abbreviation RCOO- means long chained fatty acid.

Carnitine can be isolated and purified from the muscles of a horseshoe crab and rat epididymis fluid, which have shown the biggest carnitine contents in animals. The biggest contents in mammals are in cardiac and skeletal muscle. There is little carnitine in plants. Carnitine occurs mostly as acetyl and isovaleryl carnitine. The occurence of acetyl and isovaleryl carnitine as large amounts is a part of the isolation or concentration mechanism of the transportation of carnitine. Acetyl and isovaleryl carnitine has to be hydrolyzed, before the use thereof in a cream composition according to the invention. Carrier proteins transferring carnitine through the cell membrane and acting on the membranes have been shown in studies made recently. The transportation of carnitine into the different types of cells shows both active and passive transportation.

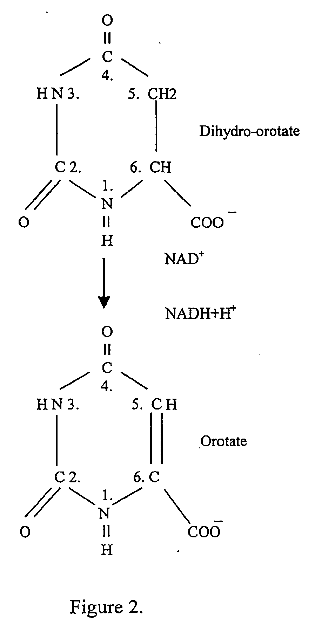

Orotic acid is an essential starting material in the biosynthesis of both RNA- and DNA - pyrimidines, and therefore a very central factor in the reproduction and action of cells. Orotate is formed in the cells from dihydro-orotate in such a way, that the hydrogen atoms are transferred from positions C-5 and C-6 to the nicotine amide adenine dinucleotide (NAD⁺) providing the generation of a double linking, whereby an orotate is formed (Figure 2.) The reaction is catalyzed by a mitochondrial dihydro-orotate dehydrogenase enzyme. All the other enzymes in the biosynthesis of pyrimidines are cytosolic.

If the mitochondria of a cell are defective or the amount of them is decreased for ageing or any other reason (e.g. mold toxins or other environmental toxins), a required amount of orotate will not be formed and the action of cell will be severely disturbed, the cell will die.

If the cell itself can no longer synthesize sufficiently orotic acid because of the shortage or the functional weakness of mitochondria, the deficiency can be replaced by orotic acid brought from outside and manage to make the injured cell recover and act normally. In respect of skin cells this means that the skin remains vital longer as well as that unhealthy skin recovers and the elasticity returns faster. Carnitine acts here as a carrier of the orotate because of its characteristics. Thus it is important to add both the vitamins into the cream composition in a right proportion and in sufficient concentration without losing other important characteristics of the composition.

The tests made with liver cell cultures have shown, that L-carnitine is able to prevent cell deaths. The effect is based on the fact that L-carnitine is able to prevent a change in permeability of the mitochondrial membrane caused by the inhibitors of the electron transportation. At the same time it was shown that the effect of L-carnitine did not require ATP i.e. energy and it did not cause a loss in the membrane potential of the mitochondrion i.e. it did not change the membrane potential. The carnitine included in a cream composition according to the invention protects cell membranes by maintaining the balance between the deacylation and reacylation activity. Although the vitamins B_{T} and B₁₃ included in the cream composition are synthetic, they are in a natural form i.e. carnitine is a pure L-form, whereby the unwanted effects caused by the D-and D, L-forms avoided.

The present invention relates to a cosmetic and dermatological cream composition improving the absorption of active ingredients containing vitamins B₁₃ i.e. orotic acid and B_{T} i.e. L-carnitine together in a basic cream composition, which contains generally accepted agents used in dermatological products. A light cream-type, oil-in-water emulsion basic cream composition may contain arachis oil, isopropyl myristate, glycerol, glycerol monostearate, methyl parahydroxy benzene, propyl parahydroxy benzene and about 70 % water. A cream-type, oil-in-water - emulsion basic cream composition may contain glycerol monostearate, glycerol, decylester of olein acid, isopropyl myristate, methyl parahydroxy benzene, propyl parahydroxy benzene and about 70 % water. A greasy, soft, water-in-oil-emulsion basic cream composition may contain cera alba, neutral plant oil, glycerol, alkylester of olein acid, isopropyl myristate, methyl parahydroxy benzene, propyl parahydroxy benzene and water.

A cream composition according to the invention contains vitamins B₁₃ and B_{T} in a basic cream composition so that the concentration of the both is between 1 - 10 %. Preferably the cream composition may contain vitamins B₁₃ and B_{T} such an amount that the concentration thereof corresponds to the maximum concentrations present in the tissues and fluids of the body.

A cream composition according to the invention contains vitamins B₁₃ and B_{T} so that the concentration of B₁₃ is at least 0,1 % and the concentration of B_{T} at least 0,2 %.

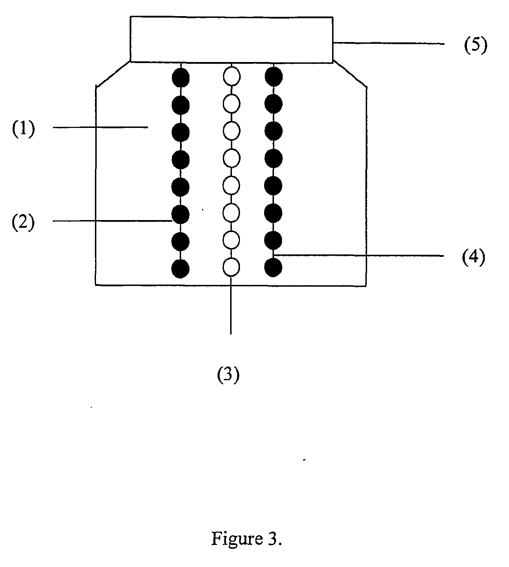

In a preferred embodiment of the invention, disclosed in Figure 3, cream compositions containing B₁₃ (1) and B_{T} (2) are as separate chaplet-like rows of balls preferably of different colors, which are provided by injection into a gel (1) in a same covered (5) package or container. When the cream composition (1) and (2) is injected into the gel (1) it forms cream composition balls having the smallest possible volume. The trace of an injection needle is shown as a thin string (4) between the balls.

The present invention relates also to a method of preparing a dermatological cream composition improving the absorption of the active ingredients by adding vitamins B₁₃ and B_{T} to a same cream composition, which contains optional agents generally used in dermatological products. If the effect of the product is wanted to increase, the cream composition can be concentrated in respect of carnitine B_{T} and orotic acid B₁₃. Preferably, the cream compositions of different colors containing B₁₃ (1) and B_{T} (2) are injected into a gel (1) in a covered (5) package in for example a can or a container.

Furthermore the invention relates to a method of improving the absorption of active ingredients, from a cosmetic or dermatological cream composition by bringing the cream composition including B₁₃ and B_{T} in contact with the skin to be treated.

The present invention relates to a method wherein the cream compositions including B₁₃ and B_{T} are applied to the skin or the skin area to be treated simultaneously in order best suitable for the transportation of the active ingredients.

A cream composition according to the invention may be used as a cosmetic product to retain the skin vital longer or in the treatment of dermatological conditions, preferably in the treatment of e.g. atopic rashes or leg ulcers. The cream composition according to the invention may also be used e.g. as a product retarding the ageing of skin and the formation of wrinkles, for a dry or irritated skin, for protecting from the sun, cold or wind.

Cream composition according to the invention is preferably used continuously on the area to be treated. The cream composition is applied on the area to be treated as needed for example once a day. In case of more severe skin problems cream composition may be applied several times during a day. The concentration of the vitamins may be defined separately according to what is necessary for the target of the use.

Next the use of the invention is illustrated in the examples which should not be considered as limiting the invention. It is obvious for a skilled person that the invention may be applied for other purposes.

### Example 1

A cream composition according to the invention was used in preparatory tests in treatment of the children's atopic skin rashes. After the use for seven days skin returned normal. The use of the cream composition was continuous, the cream composition was applied on the skin once a day.

### Example 2

A cream composition according to the invention was used in the treatment of leg ulcers occurring by the elderly. The ulcer recovering effect of the cream composition occurred already after four days of beginning of the treatment. The use was daily, the application on the skin was made 2 to 3 times a day.

## Claims

1. Cosmetic and dermatological cream composition improving the absorption of the active ingredients, **characterized in that** it contains vitamins B₁₃ i.e. orotic acid and B_{T} i.e. L-carnitine in a basic cream composition, which contains optional agents generally used in dermatological products.

2. A cream composition according to claim 1, **characterized in that** it contains vitamins B₁₃ i.e. orotic acid and B_{T} i.e. L-carnitine in a basic cream composition such amounts that the concentration of the both of them is between 1 - 10 %.

3. A cream composition according to claim 1, **characterized in that** it contains vitamins B₁₃ i.e. orotic acid and B_{T} i.e. L-carnitine so that the concentration thereof corresponds to the concentrations present in the tissues and fluids of the body.

4. A cream composition according to claim 1, **characterized in that** it contains vitamins B₁₃ i.e. orotic acid and B_{T} i.e. L-carnitine so that the concentration thereof is at least 0,1 % orotic acid and at least 0,2 % L-camitine.

5. Cosmetic and dermatological cream composition improving the absorption of the active ingredients, **characterized in that** it contains vitamins B₁₃ i.e. orotic acid and B_{T} i.e. L-carnitine in a basic cream composition, which contains optional agents generally used in dermatological products, and B₁₃ i.e. orotic acid and B_{T} i.e. L-carnitine are as separate chaplet-like balls in a gel in the same package.

6. A method of preparing a dermatological cream composition improving the absorption of the active ingredients, **characterized in that** vitamins B₁₃ i.e. orotic acid and B_{T} i.e. L-carnitine are added together into a same basic cream composition, which includes optional agents generally used in dermatological products.

7. A method of preparing a dermatological cream composition according to claim 6 **characterized in that** vitamins B₁₃ i.e. orotic acid and B_{T} i.e. L-carnitine are added together into a same basic cream composition, which includes optional agents generally used in dermatological products, and said cream composition containing B₁₃ i.e. orotic acid and B_{T} L-carnitine is injected into a gel in a package.

## Patentansprüche

1. Kosmetische und dermatologische Cremezusammensetzung, die die Absorption der Wirkstoffe verbessert, **dadurch gekennzeichnet, dass** sie die Vitamine B₁₃, d.h. Orotsäure, und B_{T}, d.h. L-Carnitin, in einer Basiscremezusammensetzung enthält, die optionale, allgemein in dermatologischen Produkten verwendete Mittel enthält.

2. Cremezusammensetzung gemäss Anspruch 1, **dadurch gekennzeichnet, dass** sie die Vitamine B₁₃, d.h. Orotsäure, und B_{T}, d.h. L-Carnitin, in einer Basiscremezusammensetzung in solchen Mengen enthält, dass die Konzentration von beiden zwischen 1 und 10 % beträgt.

3. Cremezusammensetzung gemäss Anspruch 1, **dadurch gekennzeichnet, dass** sie die Vitamine B₁₃, d.h. Orotsäure, und B_{T}, d.h. L-Carnitin, so enthält, dass ihre Konzentration der in den Geweben und Flüssigkeiten des Körpers vorhandenen Konzentration entspricht.

4. Cremezusammensetzung gemäss Anspruch 1, **dadurch gekennzeichnet, dass** sie die Vitamine B₁₃, d.h. Orotsäure, und B_{T}, d.h. L-Carnitin, so enthält, dass ihre Konzentration zumindest 0,1 % Orotsäure und zumindest 0,2 % L-Carnitin beträgt.

5. Kosmetische und dermatologische Cremezusammensetzung, die die Absorption der Wirkstoffe verbessert, **dadurch gekennzeichnet, dass** sie die Vitamine B₁₃, d.h. Orotsäure, und B_{T}, d.h. L-Carnitin, in einer Basiscremezusammensetzung enthält, die optionale, allgemein in dermatologischen Produkten verwendete Mittel enthält, und B₁₃, d.h. Orotsäure, und B_{T}, d.h. L-Carnitin, als getrennte kranzähnliche Kugeln in einem Gel in derselben Packung vorliegen.

6. Verfahren zur Herstellung einer dermatologischen Cremezusammensetzung, die die Absorption der Wirkstoffe verbessert, **dadurch gekennzeichnet, dass** die Vitamine B₁₃, d.h. Orotsäure, und B_{T}, d.h. L-Carnitin, zusammen in dieselbe Basiscremezusammensetzung gegeben werden, die optionale, allgemein in dermatologischen Produkten verwendete Mittel einschliesst.

7. Verfahren zur Herstellung einer dermatologischen Cremezusammensetzung gemäss Anspruch 6, **dadurch gekennzeichnet, dass** die Vitamine B₁₃, d.h. Orotsäure, und B_{T}, d.h. L-Carnitin, zusammen in dieselbe Basiscremezusammensetzung gegeben werden, die optionale, allgemein in dermatologischen Produkten verwendete Mittel einschliesst, und dass die Cremezusammensetzung, enthaltend B₁₃, d.h. Orotsäure, und B_{T}, d.h. L-Carnitin, in ein Gel in einer Packung injiziert wird.

## Revendications

1. Composition de crème cosmétique et dermatologique améliorant l'absorption des matières actives, **caractérisée en ce qu'**elle contient les vitamines B₁₃ à savoir l'acide orotique et B_{T} à savoir la L-carnitine dans la composition de crème de base, laquelle contient des agents optionnels utilisés de manière générale dans des produits dermatologiques.

2. Une composition de crème selon la revendication 1, **caractérisée en ce qu'**elle contient les vitamines B₁₃ à savoir l'acide orotique et B_{T} à savoir la L-carnitine dans une composition de crème de base dans des quantités telles que la concentration de celles-ci est entre 1 et 10%.

3. Une composition de crème selon la revendication 1, **caractérisée en ce qu'**elle contient les vitamines B₁₃ à savoir l'acide orotique et B_{T} à savoir la L-carnitine de manière que la concentration de celles-ci correspond aux concentrations présentes dans les tissus et les fluides du corps.

4. Une composition de crème selon la revendication 1, **caractérisée en ce qu'**elle contient les vitamines B₁₃ à savoir l'acide orotique et B_{T} à savoir la L-carnitine de manière que la concentration de celles-ci soit d'au moins 0,1% d'acide orotique et d'au moins 0,2% de L-carnitine.

5. Une composition de crème cosmétique et dermatologique améliorant l'absorption des matières actives, **caractérisée en ce qu'**elle contient les vitamines B₁₃ à savoir l'acide orotique et B_{T} à savoir la L-carnitine dans une composition de crème de base, laquelle contient des agents optionnels utilisés de manière générale dans des produits dermatologiques, et **en ce que** B₁₃ à savoir l'acide orotique et B_{T} à savoir la L-carnitine sont des billes séparées en forme de chapelet dans un gel contenu dans le même conditionnement.

6. Un procédé de préparation d'une composition de crème dermatologique améliorant l'absorption des matières actives, **caractérisée en ce que** les vitamines B₁₃ à savoir l'acide orotique et B_{T} à savoir la L-carnitine sont ajoutées ensemble dans la même composition de crème de base, laquelle comprend des agents optionnels utilisés de manière générale dans des produits dermatologiques.

7. Un procédé de préparation d'une composition de crème dermatologique selon la revendication 6, **caractérisé en ce que** les vitamines B₁₃ à savoir l'acide orotique et B_{T} à savoir la L-carnitine sont ajoutées ensemble dans la même composition de crème de base, laquelle comprend des agents optionnels utilisés de manière générale dans des produits dermatologiques, et **en ce que** ladite composition de crème contenant B₁₃ à savoir l'acide orotique et B_{T} à savoir la L-carnitine est injectée dans un gel contenu dans un conditionnement.
